# EUROPÄISCHE PATENTSCHRIFT

(11) **EP 1 654 997 B1**
(45) Veröffentlichungstag und Bekanntmachung des Hinweises auf die Patenterteilung: **12.12.2007**
(21) Anmeldenummer: 04026471.5
(22) Anmeldetag: 08.11.2004
(51) Int. Cl.: A61B 19/00, A61F 2/36

(54) **Beinlängenanpassung bei der Hüftprothesen-Chirurgie**
Adjustment of leg length in hip replacement surgery
Adaptation de la longueur de la jambe dans la pose d'une prothèse de la hanche

(43) Veröffentlichungstag der Anmeldung: 10.05.2006
(73) Patentinhaber: BrainLAB AG, 85551 Kirchheim/Heimstetten (DE)
(72) Erfinder: Tuma, Gregor, 81675 München (DE)
(74) Vertreter: Schwabe - Sandmair - Marx

(56) Entgegenhaltungen:
- EP-A- 1 417 941
- EP-A- 1 426 023
- DE-A1- 10 306 793
- US-A- 5 995 738

## Beschreibung

Die vorliegende Erfindung bezieht sich auf eine Vorrichtung und ein Verfahren zum Auswählen mindestens eines Hüftgelenkprothesenelementes aus einer Mehrzahl von Hüftgelenkprothesenelementen, insbesondere bei Hüftgelenkoperationen, wobei das mindestens eine Hüftgelenkprothesenelement basierend auf der Kontur des Beckenknochens und/oder des Oberschenkelknochens ausgewählt wird, um Beinlängenunterschiede und Beinversatz zu berücksichtigen und/oder auszugleichen.

Bei einem bekannten Verfahren zur Auswahl mindestens eines Hüftgelenkprothesenelementes bzw. einer Hüftgelenkprothese wird vor einer Operation anhand einer Röntgenaufnahme der zu operierenden Hüfte vom Operateur eine Zeichnung angefertigt. Basierend auf dieser Zeichnung werden das Modell und die Größe der Hüftgelenkprothese ausgewählt.

Die US 5,995,738 offenbart eine Vorrichtung und ein Verfahren zum Bestimmen einer Implantatposition einer künstlichen Komponente in einem Gelenk, wobei ein Gelenkmodell eines Patienten-Gelenks, in welches die künstliche Komponente implantiert werden soll, und ein Komponentenmodell der künstlichen Komponente erzeugt wird. Diese Modelle werden verwendet, um die Bewegung des Gelenks des Patienten mit der künstlichen Komponente in einer Testposition zu simulieren. Das Komponentenmodell und das Gelenkmodell werden verwendet, um einen Bewegungsbereich in dem Gelenk für mindestens eine Testposition basierend auf der simulierten Bewegung zu berechnen. Eine Implantatposition einschließlich einer Winkelausrichtung wird in dem Gelenk des Patienten basierend auf einem vorbestimmten Bewegungsbereich und dem berechneten Bewegungsbereich bestimmt.

Die EP 1 426 023 A1 offenbart ein Verfahren zur Bestimmung von Implantationsparametern für ein orthopädisches Implantat, in welchem ausgehend von den Bilddaten einer Implantationsstelle Knochendaten eines zu behandelnden Knochens berechnet werden. Weiter umfasst das Verfahren eine biomechanische Analyse eines Systems bestehend aus dem zu behandelnden Knochen und dem Implantat. In der biomechanischen Analyse werden physikalische Systemgrößen berechnet, welche anschließend mit gespeicherten Vergleichwerten verglichen werden. Der Vergleich ermöglicht eine Bewertung des durchgerechneten Systems. Die Daten des Systems stehen anschließend als Implantationsparameter zur Weiterverarbeitung zur Verfügung.

Es ist eine Aufgabe der vorliegenden Erfindung eine Vorrichtung zum Auswählen mindestens eines Hüftgelenkprothesenelementes aus einer Mehrzahl von Hüftgelenkprothesenelementen vorzuschlagen, mit welchen eine Erhöhung der Lebensdauer von Hüftgelenkprothesen erreicht werden kann.

Diese Aufgabe wird durch den Gegenstand des unabhängigen Anspruchs gelöst. Vorteilhafte Ausführungsformen ergeben sich aus den Unteransprüchen.

Um mindestens ein Hüftgelenkprothesenelement aus einer Mehrzahl an Hüftgelenkprothesenelementen auszuwählen, umfasst eine erfindungsgemäße Vorrichtung zum Auswählen des mindestens einen Hüftgelenkprothesenelements vorzugsweise ein Datenerfassungsgerät, gemäß Anspruch 1.

Vorzugsweise kann das erfindungsgemäße Datenerfassungssystem mindestens einen mit Markern versehenen Pointer und mindestens einer Kamera, insbesondere eine Infrarot-Kamera, und/oder Infrarot-Lampen umfassen, wobei die Marker insbesondere Infrarotstrahlung emittierende aktive Marker oder Infrarotstrahlung reflektierende passive Marker sein können, die durch die mindestens eine Kamera detektiert werden können, so dass auf die Position des Pointers und damit auf die Kontur des Beckenknochens und/oder des Oberschenkelknochens geschlossen werden kann. Das Datenerfassungssystem kann auch ein mit Markern versehenes Instrument zur Entfernung von Knochen- und/oder Knorpelmaterial umfassen, welches auch von der Kamera detektiert werden kann, woraus auf die Position des Instruments und damit auf die Menge des entfernten Knochen- und/oder Knorpelmaterials geschlossen werden kann. Die Kontur oder die Form des Beckenknochens und/oder des Oberschenkelknochens kann auch zum Beispiel mit einem Computertomographen und/oder einem Kernspin-Tomographen und/oder einem Ultraschall-Tomographen und/oder einem Positronen-Emissions-Tomographen und/oder einem SPECT-Tomographen erfasst oder registriert werden, welche auch zu dem Datenerfassungssystem gehören können.

Vorzugsweise umfasst die Vorrichtung zum Auswählen mindestens eines Hüftgelenkprothesenelementes aus einer Mehrzahl an Hüftgelenkprothesenelementen eine Datenausgabevorrichtung, insbesondere einen Bildschirm, der die Knochen- und/oder Knorpelkonturdaten, zum Beispiel als Zahlenwerte, und/oder das ausgewählte Hüftgelenkprothesenelement, zum Beispiel eine das jeweilige Hüftgelenkprothesenelement kennzeichnende Nummer oder das Hüftgelenkprothesenelement selbst in grafischer Darstellung, ausgeben kann. Auch kann eine Dateneingabevorrichtung insbesondere eine Tastatur und/oder ein Scanner wie ein 3D-Scanner vorgesehen sein, die zum Beispiel mit der Recheneinheit oder der Datenbank oder dem Speicher verbunden sein kann. Mit der Dateneingabevorrichtung können zum Beispiel Daten, wie Daten von verschiedenen bekannten Hüftgelenkprothesenelementen, eingegeben werden oder bekannte Hüftgelenkprothesenelemente selbst eingescannt werden und zum Beispiel in der Datenbank oder dem Speicher abgespeichert werden und/oder es können Daten, wie zum Beispiel erfasste Knochen- und/oder Knorpelkonturdaten, eingegeben werden, die in der Recheneinheit verarbeitet werden können und anschließend mit bekannten Daten der Datenbank verglichen werden können.

Insbesondere kann die erfindungsgemäße Vorrichtung ein Navigationssystem umfassen, welches zum Beispiel mit der Recheneinheit verbunden sein kann und zum Beispiel ein Instrument zur Entfernung von Knochen- und/oder Knorpelmaterial, an dem Marker angeordnet sein können, mittels der erfassten Knochen- und/oder Knorpelkonturdaten navigieren kann, indem zum Beispiel das Navigationssystem aus der erfassten Form oder Kontur des Beckenknochens und/oder des Oberschenkelknochens und der Menge an Knochen- und/oder Knorpelmaterial, welches entfernt werden soll, ermittelt, wie das Instrument navigiert werden soll oder an welche Positionen das Instrument geführt werden soll oder welche Bewegungen das Instrument ausführen soll.

Bevorzugt wird mit der erfindungsgemäßen Vorrichtung das ausgewählte Hüftgelenkprothesenelement automatisch an einer Stelle, wie zum Beispiel an dem Beckenknochen oder an oder in dem Oberschenkelknochen positioniert.

Das ausgewählte Hüftgelenkprothesenelement, welches sowohl eine Prothesenpfanne als auch ein Prothesenkopf oder ein mit einem Prothesenschaft verbundener Prothesenkopf sein kann, weist ein teilkugelförmiges Oberflächenelement auf. Das Oberflächenelement ist zumindest nahezu halbkugelförmig oder ist ein Kugelsegment bzw. ein Kugelsektor oder kann als Kugelsegment oder Kugelsektor einer Hohlkugel bezeichnet werden, wobei die Außenkontur und die Innenkontur des Oberflächenelements teilkugelförmig oder zumindest nahezu halbkugelförmig sein kann. Durch Einsetzen eines Zusatzelementes zum Beispiel in die Innenkontur des Prothesenkopfes oder auf die Außenkontur der Prothesenpfanne kann der Durchmesser bzw. der Radius der Innenkontur oder der Außenkontur des Hüftgelenkprothesenelementes variiert werden. Wird das Zusatzelement in die Innenkontur des Prothesenkopfes eingesetzt, so wird der Durchmesser der konvexen Innenkontur des Prothesenkopfes um die zweifache Dicke des Zusatzelementes verringert bzw. der Radius der Innenkontur des Prothesenkopfes um die Dicke des Zusatzelementes verringert. Auch kann das Zusatzelement auf die Außenkontur der Prothesenpfanne aufgesetzt werden, so dass der Durchmesser der konkaven Außenkontur der Prothesenpfanne um einen Betrag zunimmt, welcher der zweifachen Dicke des Zusatzelementes entspricht. Das Zusatzelement kann auch ein herausnehmbares Element sein, welches aus dem Hüftgelenkprothesenelement oder der Innen- oder Außenkontur des Hüftgelenkprothesenelementes entfernt werden kann, wobei durch Herausnehmen des Zusatzelementes der Durchmesser der Außen- und/oder Innenkontur des Hüftgelenkprothesenelementes variiert werden kann. Das Zusatzelement kann zum Beispiel aus der Innenkontur des Prothesenkopfes herausgenommen werden, so dass sich der Durchmesser der Innenkontur des Prothesenkopfes entsprechend der zweifachen Dicke des Zusatzelementes, welches entfernt wurde, vergrößert. Das Zusatzelement kann auch von der Außenkontur oder der Oberfläche der Prothesenpfanne abgenommen oder herausgenommen werden, so dass der Durchmesser der Außenkontur der Prothesenpfanne entsprechend dem Doppelten der Dicke des Zusatzelementes verringert wird. Das Zusatzelement kann verschiedene Dicken aufweisen. Es kann zum Beispiel einen oder drei oder fünf mm dick sein, oder es kann bis zu 20 mm dick sein.

Durch das Einsetzen oder Herausnehmen des Zusatzelementes können insbesondere Beinlängenunterschied oder ein Beinversatz ausgeglichen werden. So kann zum Beispiel mit der Vorrichtung der vorliegenden Erfindung ein Hüfgelenkprothesenelement ausgewählt werden, welches zum Ausgleichen oder Herstellen eines bestehenden oder ermittelten oder gewünschten Beinlängenunterschieds oder Beinversatzes mit einem Zusatzelement versehen sein oder werden kann.

Auch kann das Zusatzelement in den Prothesenschaft, der zum Beispiel mit dem Prothesenkopf verbunden sein kann oder verbunden werden kann eingesetzt oder angebracht werden oder das Zusatzelement kann aus dem Prothesenschaft herausgenommen oder abgenommen werden, wobei die Länge des Prothesenschaftes durch Entfernen oder Hinzufügen des Zusatzelementes verändert werden kann. Zum Beispiel kann das Zusatzelement auf den Prothesenschaft aufgesetzt werden oder in den Prothesenschaft eingesetzt werden, so dass sich die Länge des Prothesenschaftes durch das Hinzufügen des Zusatzelementes um die Länge des Zusatzelementes vergrößert oder das Zusatzelement kann von dem Prothesenschaft abgenommen werden oder aus dem Prothesenschaft herausgenommen werden, so dass sich die Länge des Prothesenschaftes um die Länge des Zusatzelementes verringert. Das Zusatzelement kann einen oder drei oder fünf oder mehr mm lang sein oder es kann bis zu 20 mm lang sein.

Die Erfindung wird nachfolgend anhand bevorzugter Ausführungsbeispiele beschrieben werden. Es zeigen:
- Figur 1: eine Vorrichtung gemäß einer ersten Ausführungsform der Erfindung sowie eine Hüfte 1 und einen Oberschenkelknochen 3;
- Figur 2: eine Hüfte 4 und einen Oberschenkelknochen 3 vor und nach Entfernen von Knochen- und /oder Knorpelmaterial;
- Figur 3: einen Oberschenkelknochen 3 vor und nach Entfernen von Knochen- und/oder Knorpelmaterial und eine Hüfte 4 vor und nach Entfernen von Knochen- und/oder Knorpelmaterial sowie Instrumente 22, 23 zum Entfernen von Knochen- und/oder Knorpelmaterial;
- Figur 4A: einen Oberschenkelknochen 3 mit eingesetzter Prothese;
- Figur 4B: eine Hüftgelenkprothese,
- Figur 5: Ablaufdiagramm einer Ausführungsform des Verfahrens, welches mit der erfindungsgemäßen Vorrichtung ausgeführt wird

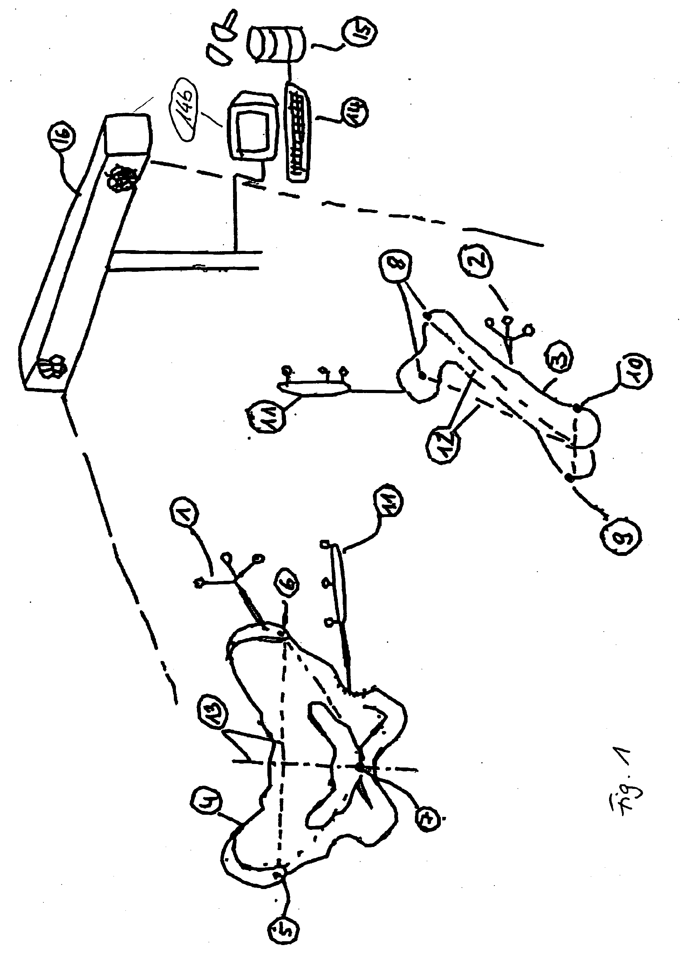

Figur 1 zeigt eine Vorrichtung zum Auswählen eines Hüftgelenkprothesenelementes aus einer Mehrzahl von Hüftgelenkprothesenelementen mit einem Navigationssystem 14, einem Bildschirm 14b, einer Datenbank 15 und einer Kamera 16, zum Beispiel einer Infrarot-Kamera. An einer Hüfte 4 und an einem Oberschenkelknochen 3 ist jeweils ein Referenzstern 1, 2 angebracht, wodurch die Hüfte 4 und der Oberschenkelknochen 3 mittels der Kamera 16 erfasst und registriert werden können, so dass dem Navigationssystem 14 die Position der Hüfte 4 und des Oberschenkelknochens 3 also deren genaue Lage im Raum bekannt ist. Insbesondere können die Hüfte 4 und der Oberschenkelknochen 3 mit Hilfe bekannter Punkte, wie Landmarken 5, 6, 7, 8, 9, 10 registriert werden, indem zum Beispiel mit einem Pointer 11, an welchem auch Marker befestigt sind, die Oberfläche der Hüfte 4, insbesondere der Hüftgelenkpfanne und des Oberschenkelknochens 3, insbesondere des Oberschenkelknochenkopfes, zum Beispiel mit der Spitze des Pointers abgefahren und detektiert und registriert werden oder mit einem Pointer 11 optisch, zum Beispiel mittels eines Lasers, abgetastet und detektiert und registriert werden. In der Recheneinheit der erfindungsgemäßen Vorrichtung kann aus den erfassten Daten, wie den Knochen- und/oder Knorpelkonturdaten oder der Form der Hüfte 4 und/oder des Oberschenkelknochens 3, eine neutrale Stehposition eines Menschen und/oder ein vorgegebener Beinlängenunterschied und/oder ein vorgegebener Beinversatz errechnet werden. Insbesondere wird die Hüftgelenkpfanne und/oder der Kopf des Oberschenkelknochens mit dem Pointer 11 registriert, um in der Recheneinheit und/oder in dem Navigationssystem 14 die genaue Form oder Kontur der Hüftgelenkpfanne und/oder des Kopfes des Oberschenkelknochens 3 digital abbilden zu können und so eine neutrale Position der Hüfte 4 und des Oberschenkelknochens 3 zueinander oder eine neutrale Stehposition eines Menschen oder einen vorgegebenen Beinlängenunterschied und/oder einen vorgegebenen Beinversatz simulieren zu können. Mit Hilfe der, zum Beispiel durch die Simulation, gewonnen Daten kann nun zum Beispiel das Navigationssystem 14 ein Instrument, auf dem auch Marker angebracht sein können, so navigieren, dass eine vorgegebene Menge an Knochen- und/oder Knorpelmaterial von dem Beckenknochen und/oder von dem Oberschenkelknochen entfernt wird.

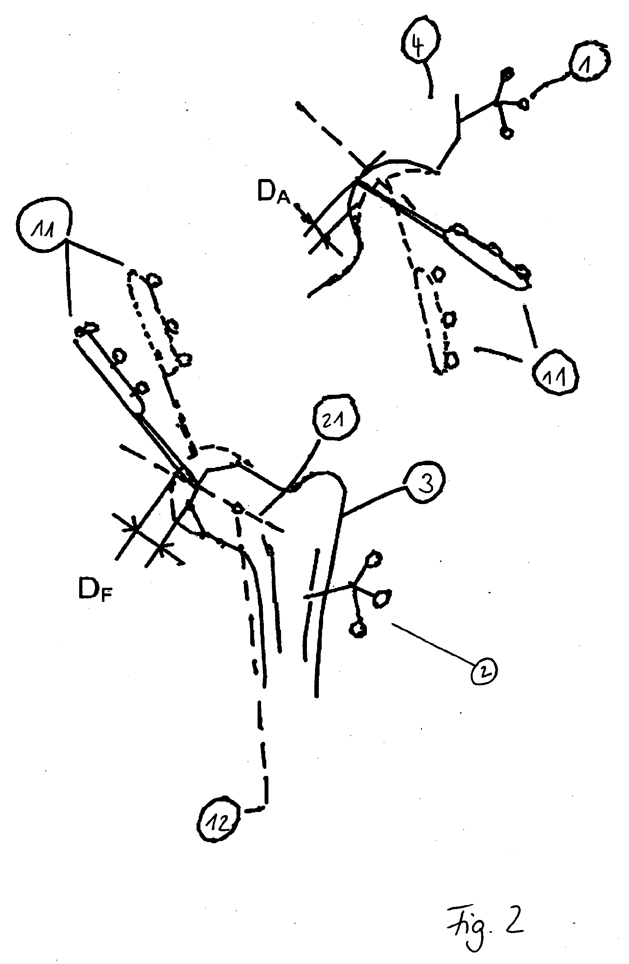

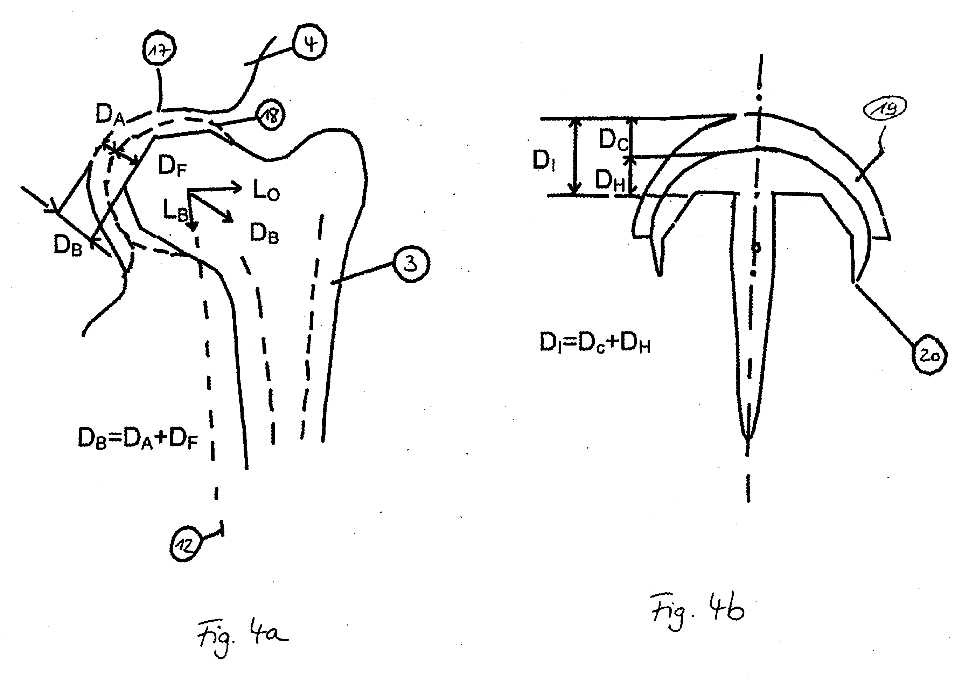

Figur 2 zeigt eine Hüfte 4 und einen Oberschenkelknochen 3, insbesondere eine Hüftgelenkpfanne der Hüfte 4 und den Kopf des Oberschenkelknochens 3, auf denen Marker 1, 2 angeordnet sind und deren Kontur oder Oberfläche mit einem Pointer 11 registriert wird. Gestrichelt ist dabei der Pointer 11 bei der Registrierung der ursprünglichen Form der Hüftgelenkpfanne und des Kopfes des Oberschenkelknochens 3 zu erkennen, sowie die ursprüngliche Kontur der Hüfte 4 bzw. der Hüftgelenkpfanne und des Oberschenkelknochens 3 bzw. des Kopfes des Oberschenkelknochens 3. Des Weiteren zeigt Figur 2 den Kopf des Oberschenkelknochens 3 nach dem Entfernen von Knochen- und/oder Knorpelmaterial sowie die Differenz D_{F} zwischen der ursprünglichen Kontur des Oberschenkelknochenkopfes und dessen Kontur nach Entfernen des Knochen- und/oder Knorpelmaterials und den Pointer 11 bei der Registrierung der neuen Kontur. Ebenso zeigt Figur 2 die neue Kontur der Hüftgelenkpfanne der Hüfte 4 nach dem Entfernen von Knochen- und/oder Knorpelmaterial sowie den Unterschied D_{A} zwischen der ursprünglichen und der neuen Kontur der Hüftgelenkpfanne und den Pointer 11 während des Registrierens der neuen Kontur oder der neuen Form der Hüftgelenkpfanne nach dem Entfernen von Knochen- und/oder Knorpelmaterial. Die Differenzen D_{F} und D_{A} können zum Beispiel drahtgebunden oder drahtlos zum Beispiel von dem Datenerfassungssystem an die Recheneinheit und/oder an das Navigationssystem 14 übertragen werden, worin diese Daten weiterverarbeitet werden können und mit bekannten Knochen und/oder Knorpelkonturdaten, wie bekannten Differenzen D_{F} und D_{A}, die zum Beispiel in der Datenbank 15 gespeichert sein können, verglichen werden, um mindestens ein geeignetes Hüftgelenkprothesenelement aus der Datenbank 15 auszuwählen. Bei dem Vergleich können zum Beispiel die Hüftgelenkprothesenelemente ausgewählt werden, die das entfernte Knochen- und/oder Knorpelmaterial ersetzen oder approximieren oder nachbilden. Insbesondere kann zum Beispiel eine Prothesenpfanne 19 in Figur 4b, ausgewählt werden, deren Dicke D_{C} die Dicke D_{A} des entfernten Knochen- und/oder Knorpelmaterials der Hüfte 4 bestmöglich approximiert oder deren Dicke D_{C} der Dicke D_{A} zumindest nahezu entspricht. Vorzugsweise wird der Prothesenkopf 20 mit zugehörigem Prothesenschaft ausgewählt, wobei die Dicke D_{H} des Prothesenkopfes 20 der Dicke D_{F} des von dem Oberschenkelknochen 3 entfernten Knochen- und/oder Knorpelmaterials zumindest nahezu entspricht oder mit dieser zumindest nahezu übereinstimmt oder diese approximiert.

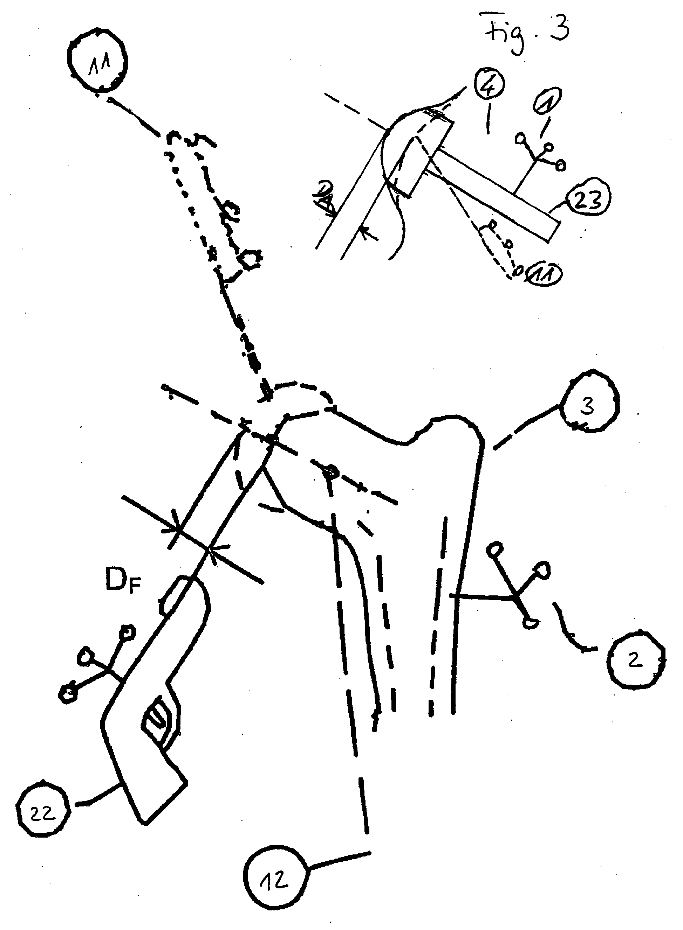

In Figur 3 werden Instrumente 22, 23 zur Entfernung von Knochen- und/oder Knorpelmaterial gezeigt, die jeweils mit Markern, wie einem Referenzstern, versehen sein können und so zum Beispiel durch das Navigationssystem 14 navigiert und/oder positioniert werden können. Dabei können zum Beispiel Daten oder Informationen über eine bestimmte Menge an Knochen- und/oder Knorpelmaterial dem Navigationssystem 14 übergeben werden oder diesem bekannt sein, so dass mit Hilfe des Navigationssystems 14 die Instrumente 22, 23 so navigiert oder bewegt werden können, dass die gewünschte Menge an Knochen- und/oder Knorpelmaterial entfernt werden kann, um bestimmte gewünschte oder eingegebene Differenzen D_{F} und D_{A} zwischen der alten und der neuen Kontur der Hüftgelenkpfanne und/oder der alten und der neuen Kontur des Oberschenkelknochenkopfes zu erreichen. Die Instrumente 22, 23 können auch so bewegt werden, dass Differenzen D_{F} und D_{A} erreicht werden, die auf Grund individueller anatomischer Gegebenheiten, wie einem bestimmten Körperbau, oder medizinischen Gegebenheiten notwendig sind, um für den Patienten zum Beispiel eine gesundheitliche Verbesserung zu ermöglichen. Insbesondere können die Instrumente 22, 23 von einem Operateur oder automatisch mit Hilfe der Daten des Navigationssystems 14 bewegt werden.

Die Recheneinheit kann zum Beispiel durch Vergleich der erfassten Knochen- und/oder Knorpelkonturdaten mindestens ein Hüftgelenkprothesenelement, wie einen Prothesenkopf 20 und/oder ein Prothesenschaft und/oder eine Prothesenpfanne 19, aus einer Vielzahl von zum Beispiel in der Datenbank 15 gespeicherten Hüftgelenkprothesenelementen auswählen, und zum Beispiel die geeignetste Wahl, bei der zum Beispiel die Dicke D_{C} der Prothesenpfanne 19 ungefähr dem Unterschied D_{A} zwischen der alten und der neuen Kontur der Hüftgelenkpfanne entspricht und/oder bei welcher die Dicke D_{H} des Prothesenkopfes 20 ungefähr dem Unterschied D_{F} der alten und der neuen Kontur des Oberschenkelknochenkopfes entspricht, ermitteln und zum Beispiel das oder die gewählten Hüftgelenkprothesenelemente ausgeben. Solche Hüftgelenkprothesenelemente finden sich in Figur 4B, wobei die aus den Hüftgelenkprothesenelementen gebildete Hüftgelenkprothese aus einer Prothesenpfanne 19 und einem Verankerungselement mit Prothesenkopf 20 besteht. Dabei kann die gesamte Prothese, wie in Figur 4B gezeigt, zum Beispiel aus zwei Teilen, wie der Prothesenpfanne 19 und dem Verankerungselement mit Prothesenkopf 20 bestehen oder auch aus drei oder mehr Teilen bestehen, wie einer Prothesenpfanne 19, die in der Hüftgelenkpfanne gelagert und mit einem Prothesenkopf 20 verbunden werden kann, welcher wiederum mit einem Verankerungselement, wie einem Prothesenschaft, verbunden werden kann, welches in den Oberschenkelknochen eingebracht, wie zum Beispiel einzementiert, werden kann. Figur 4A zeigt den Oberschenkelknochen 3, sowie die Prothese und die Hüfte 4 nach einer Operation, wobei der Oberschenkelknochen 3 und die Hüfte 4 so verbunden sind, dass die Differenz D_{F} der Kontur des Oberschenkelknochens 3 vor und nach dem Entfernen von Knochen und/oder Knorpelmaterial durch den Prothesenkopf 20 zumindest nahezu vollständig kompensiert wird und der Unterschied der Hüfte 4 oder Hüftgelenkpfanne vor und nach dem Entfernen von Knochen und/oder Knorpelmaterial durch die Prothesenpfanne 19 zumindest nahezu vollständig kompensiert wird.

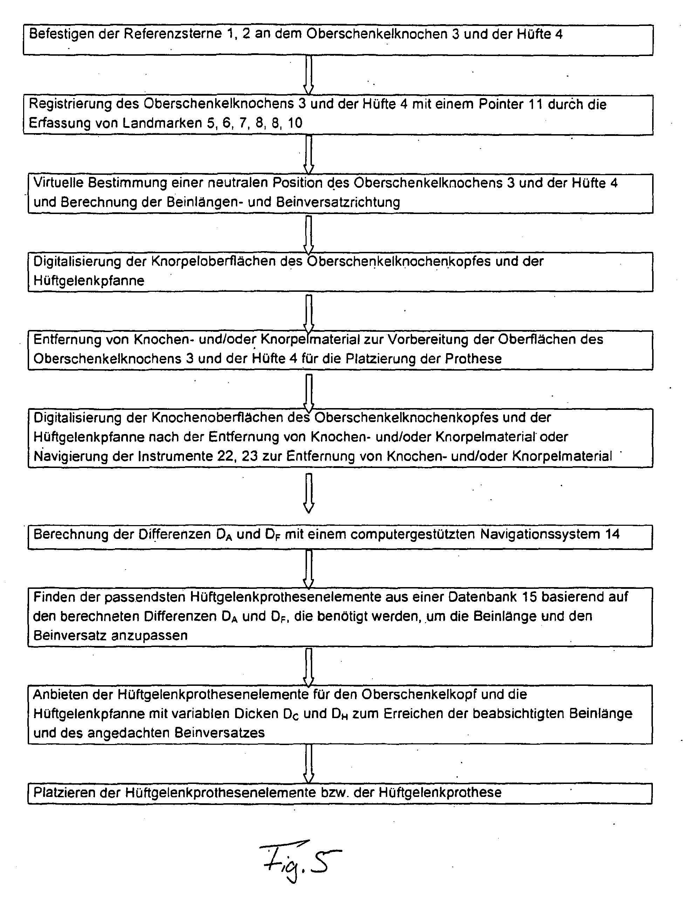

Figur 5 zeigt ein Ablaufdiagramm eine Ausführungsform des Verfahrens welches mit der erfindungsgemäße Vorrichtung ausgeführt wird. Hierbei werden in einem ersten Schritt an dem Oberschenkelknochen 3 und an der Hüfte 4 Referenzsterne 1, 2 angeordnet oder befestigt, wobei anschließend mit einem Pointer 11 Landmarkenpunkte 5, 6, 7, 8, 9, 10 erfasst werden, wodurch der Oberschenkelknochen 3 und die Hüfte 4 registriert werden. Nachfolgend wird virtuell, wie zum Beispiel in der Recheneinheit eine neutrale Position des Oberschenkelknochens 3 und der Hüfte definiert oder ermittelt und die Beinlängen- und Beinversatzrichtung berechnet. In einem folgenden Schritt werden die Knorpelkonturen oder -oberflächen des Oberschenkelknochenkopfes und der Hüftgelenkpfanne digitalisiert, worauf Knochen- und/oder Knorpelmaterial des Oberschenkelknochens 3 und der Hüfte 4 entfernt wird, um eine Platzierung der Hüftgelenkprothese zu gewährleisten. Die Entfernung des Knochen- und/oder Knorpelmaterials kann auch mit Instrumenten 22, 23 durchgeführt werden, die von dem Navigationssystem navigiert werden. Nach der Entfernung des Knochen- und/oder Knorpelmaterials wird die Knochenkontur oder -oberfläche des Oberschenkelknochenkopfes und der Hüftgelenkpfanne erneut digitalisiert, um in dem computergestützten Navigationssystem 14 die Differenzen D_{A} und D_{F} zwischen den Konturen der Hüftgelenkpfanne und des Oberschenkelknochenkopfes vor und nach der Entfernung des Knochen- und/oder Knorpelmaterials zu berechnen. Anschließend können unter Berücksichtigung der berechneten Differenzen D_{A} und D_{F} die passendsten Hüftgelenkprothesenelemente in einer Datenbank 15 gesucht werden, wobei die Hüftgelenkprothesenelemente von dem Navigationssystem oder der Recheneinheit gefunden und ausgegeben werden, deren Dicken D_{C} und D_{H} bestmöglich die berechneten Differenzen D_{A} und D_{F} approximieren und damit zum Beispiel die bisherige Beinlänge und/oder den bisherigen Beinversatz erhalten oder wiederherstellen oder die berechnete oder gewünschte Beinlänge und/oder den berechneten oder gewünschten Beinversatz herstellen. Anschließend werden die ausgegebenen oder gefundenen Hüftgelenkprothesenelemente oder die aus den ausgewählten Hüftgelenkprothesenelementen bestehende Hüftgelenkprothese, zum Beispiel manuell, wie von einem Operateur, oder automatisch, wie von einem Roboter positioniert oder implantiert, wobei die ausgewählte Prothesenpfanne in der Hüftgelenkpfanne positioniert wird und der ausgewählte Prothesenschaft und/oder der ausgewählte Prothesenkopf in dem Oberschenkelknochen platziert wird.

### Bezugszeichen

- 1, 2: Referenzstern
- 3: Oberschenkelknochen
- 4: Hüfte
- 5-10: Landmarken
- 11: Pointer
- 12: Beinlängenrichtung, Beinversatzrichtung
- 13: vordere Hüftebene, Mittsagittalebene
- 14: Navigationssystem
- 14b: Datenausgabevorrichtung, Bildschirm
- 15: Datenbank
- 16: Kamera, Infrarot-Kamera
- 17: entferntes Knochen- und/oder Knorpelmaterial der Hüfte
- 18: entferntes Knochen- und/oder Knorpelmaterial des Oberschenkelknochens
- 19: Prothesenpfanne
- 20: Prothesenkopf
- 21: Zentralachse durch den Schenkelhals des Femur- bzw. des Oberschenkelknochenkopfes, Vertikalachse
- 22, 23: Instrumente zur Entfernung von Knochen und/oder Knorpelmaterial

## Patentansprüche

1. Vorrichtung zum Auswählen mindestens eines Hüftgelenkprothesenelementes aus einer Mehrzahl an Hüftgelenkprothesenelementen mit:
- einem Datenerfassungssystem zum Erfassen eines ersten Satzes von Knochen- und/oder Knorpelkonturdaten einer Hüfte (4) und/oder eines Oberschenkelknochen (3) und zum Erfassen eines zweiten Satzes von Knochen- und/oder Knorpelkonturdaten einer Hüfte (4) und/oder eines Oberschenkelknochen (3) nach der Entfernung von Knochen- und/oder Knorpelmaterial der Hüfte (4) und/oder des Oberschenkelknochens (3),
- einer Recheneinheit zum Verarbeiten des ersten und zweiten Satzes der erfassten Knochen- und/oder Knorpelkonturdaten, wobei die Recheneinheit mit dem Datenerfassungssystem verbunden ist, und
- einer Datenbank (15), in welcher charakteristische Daten einer Mehrzahl von Hüftgelenkprothesenelementen gespeichert sind, **gekennzeichnet dadurch, dass** die Recheneinheit in Abhängigkeit von dem erfassten ersten Satz und dem erfassten zweiten Satz von Knochen- und/oder Knorpelkonturdaten mindestens ein Hüftgelenkprothesenelement aus der Datenbank (15) auswählt.

2. Vorrichtung nach dem vorhergehenden Anspruch, wobei das Datenerfassungssystem mindestens einen Pointer (11) mit Markern und mindestens eine Kamera (16), insbesondere eine Infrarot-Kamera, und/oder Infrarot-lampen und/oder ein Instrument (22, 23) mit Markern zur Entfernung von Knochen- und/oder Knorpelmaterial und/oder einen Computertomographen und/oder einen Kernspin-Tomographen und/oder einem Ultraschall-Tomographen und/oder einen Positronen-Emissions-Tomographen und/oder einen SPECT-Tomographen umfasst.

3. Vorrichtung nach einem der zwei vorhergehenden Ansprüche mit einer Datenausgabevorrichtung, insbesondere einem Bildschirm (14b), zur Ausgabe der Knochen- und/oder Knorpelkonturdaten und/oder der ausgewählten Hüftgelenkprothesenelementen und/oder mit einer Dateneingabevorrichtung, insbesondere einer Tastatur und/oder einem Scanner, zur Eingabe von Daten zur Abspeicherung in der Datenbank, wie Hüftgelenkprothesenelementdaten, und/oder Daten zur Verarbeitung in der Recheneinheit, wie Knochen- und/oder Knorpelkonturdaten.

4. Vorrichtung nach einem der drei vorhergehenden Ansprüche mit einem Navigationssystem (14), wobei das Navigationssystem (14) mindestens ein Instrument (22, 23) mit Markern zur Entfernung von Knochen- und/oder Knorpelmaterial mittels der erfassten Knochen- und/oder Knorpelkonturdaten navigieren kann.

## Claims

1. A device for selecting at least one hip joint prosthetic element from a number of hip joint prosthetic elements, comprising:
- a data detection system for detecting a first set of bone and/or cartilage contour data of a hip (4) and/or femur (3) and for detecting a second set of bone and/or cartilage contour data of a hip (4) and/or femur (3) after bone and/or cartilage material of the hip (4) and/or femur (3) has been removed;
- a computational unit for processing the first and second set of detected bone and/or cartilage contour data, wherein the computational unit is connected to the data detection system; and
- a database (15) in which characteristic data of a number of hip joint prosthetic elements are stored, **characterised in that** the computational unit selects at least one hip joint prosthetic element from the database (15) in accordance with the detected first set and detected second set of bone and/or cartilage contour data.

2. The device according to the preceding claim, wherein the data detection system comprises at least one pointer (11) comprising markers, and at least one camera (16), in particular an infrared camera, and/or infrared lamps and/or an instrument (22, 23) comprising markers, for removing bone and/or cartilage material and/or a computer tomograph and/or a nuclear spin tomograph and/or an ultrasound tomograph and/or a positron emission tomograph and/or a SPECT tomograph.

3. The device according to any one of the preceding two claims, comprising a data output device, in particular a screen (14b), for outputting the bone and/or cartilage contour data and/or the selected hip joint prosthetic element and/or comprising a data input device, in particular a keyboard and/or a scanner, for inputting data to be stored in the database, such as hip joint prosthetic element data, and/or data to be processed in the computational unit, such as bone and/or cartilage contour data.

4. The device according to any one of the preceding three claims, comprising a navigation system (14), wherein the navigation system (14) can navigate at least one instrument (22, 23) comprising markers, for removing bone and/or cartilage material, by means of the detected bone and/or cartilage contour data.

## Revendications

1. Dispositif pour sélectionner au moins un élément de prothèse de hanche parmi une pluralité d'éléments de prothèse de hanche, comportant :
- un système d'acquisition de données, pour acquérir un premier ensemble de données sur les contours de l'os et/ou les contours du cartilage d'un os iliaque (4) et/ou d'un fémur (3) et pour acquérir un deuxième ensemble de données sur les contours de l'os et/ou les contours du cartilage d'un os iliaque (4) et/ou d'un fémur (3) après l'enlèvement de la matière osseuse et/ou cartilagineuse de l'os iliaque (4) et/ou du fémur (3),
- une unité de calcul pour traiter les premier et deuxième ensembles de données sur les contours de l'os et/ou les contours du cartilage, l'unité de calcul étant reliée au système d'acquisition de données, et
- une base de données (15), dans laquelle sont mémorisées des données caractéristiques d'une pluralité d'éléments de prothèse de hanche,
**caractérisé en ce que** l'unité de calcul sélectionne dans la base de données (15) au moins un élément de prothèse de hanche en fonction du premier ensemble acquis ou du deuxième ensemble acquis de données sur les contours de l'os et/ou les contours du cartilage.

2. Dispositif selon la revendication précédente, dans lequel le système d'acquisition de données comporte au moins un pointeur (11) avec des repères et au moins une caméra (16), en particulier une caméra à infrarouge, et/ou des lampes à infrarouge et/ou un instrument (22, 23) avec des repères pour l'enlèvement de la matière osseuse et/ou cartilagineuse, et/ou un tomographe assisté par ordinateur et/ou un tomographe à résonance magnétique et/ou un tomographe à ultrasons et/ou un tomographe à émission de positons et/ou un tomographe à émission monophotonique.

3. Dispositif selon l'une des deux revendications précédentes, comportant un dispositif d'édition des données, en particulier un écran (14b) pour afficher les données sur les contours de l'os et/ou les contours du cartilage et/ou des éléments de prothèse de hanche sélectionnés et/ou comportant un dispositif d'entrée de données, en particulier un clavier et/ou un scanner, pour entrer des données à mémoriser dans la base de données, telles que des données sur les éléments de prothèse de hanche et/ou des données, telles que les données sur les contours de l'os et/ou les contours du cartilage, destinées à être traitées dans l'unité de calcul.

4. Dispositif selon l'une des trois revendications précédentes, comportant un système de navigation (14), le système de navigation (14) pouvant piloter au moins un instrument (22, 23) avec des repères pour éliminer la matière osseuse et/ou cartilagineuse au moyen des données acquises sur les contours de l'os et/ou les contours du cartilage.
